# EUROPÄISCHE PATENTSCHRIFT

(11) **EP 2 046 431 B1**
(45) Veröffentlichungstag und Bekanntmachung des Hinweises auf die Patenterteilung: **15.07.2015**
(21) Anmeldenummer: 07787540.9
(22) Anmeldetag: 13.07.2007
(51) Int. Cl.: A61M 16/04

(54) **TRACHEOSTOMIEKANÜLE MIT INNENKANÜLE**
TRACHEOSTOMY CANNULA WITH INNER CANNULA
CANULE DE TRACHEOTOMIE AVEC CANULE INTERNE

(30) Priorität: 31.07.2006 DE 102006035887
(43) Veröffentlichungstag der Anmeldung: 15.04.2009
(73) Patentinhaber: Tracoe Medical Gmbh, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2007/057272
(87) Internationale Veröffentlichungsnummer: WO 2008/015094

(56) Entgegenhaltungen:
- EP-A- 1 159 980
- EP-A- 1 407 796
- DE-A1- 10 109 935
- US-A- 2 923 299
- US-A- 3 606 669
- US-A- 4 987 895
- US-A- 5 443 064

## Beschreibung

Die vorliegende Erfindung betrifft eine Tracheostomiekanüle, bestehend aus einer Außenkanüle und einer darin einsetzbaren Innenkanüle. Viele Patienten und Ärzte bevorzugen derartige Tracheostomiekanülen mit Innenkanüle, die schon seit längerem in der Praxis gebraucht werden. Die Innenkanülen solcher zweilumigen Tracheostomiekanülen können problemlos entnommen und ausgetauscht werden. Auf diese Weise können Verkrustungen bzw. Verborkungen an der Kanülenwand einfacher und leichter entfernt werden, da die Innenkanüle ohne Kontakt mit der Oberfläche der Trachea oder den Wänden des Tracheostoma aus der Außenkanüle herausgenommen und wieder eingeschoben werden kann und die Innenkanüle dann außerhalb des Körpers gereinigt werden kann. Beispiele sind offenbart in DE 10109935 und EP 1159980.

Die Reinigung von einlumigen Kanülen (d.h. Tracheostomiekanülen ohne Innenkanüle) kann dagegen für ungeübte Patienten bzw. Pfleger ein Problem darstellen. Je nach Verunreinigung kann es vorkommen, daß die Kanüle zwecks Reinigung entnommen werden muß, da eine Absaugung nicht ausreichend ist und bei der im Tracheostoma verbleibenden Kanüle auch nur schwierig und nur mit erheblichen Unannehmlichkeiten für den Patienten möglich ist. Die Entnahme der (einlumigen) Kanüle zwecks Reinigung kann jedoch mit erheblichem Risiko verbunden sein. Da sich das Stoma in vielen Fällen nach Entnahme der Kanüle zusammenzieht, kann es vorkommen, daß der Patient unter Atemnot leidet. Die Wiedereinführung einer Kanüle in solch ein enges Stoma gestaltet sich in vielen Fällen als sehr schwierig. Es droht Erstickungsgefahr.

Die Verwendung von zweilumigen Tracheostomiekanülen hat dagegen den Vorteil, daß die Reinigung der Innenkanüle aus den oben genannten Gründen wesentlich einfacher ist. Zusätzlich besteht die Möglichkeit, daß ein Patient bei sogenannten "gefensterten" Sprechkanülen wahlweise eine Innenkanüle mit oder ohne Fenestrierung einsetzen und diese nach Belieben relativ schnell und einfach austauschen kann. Eine ebenfalls mit Fenster versehene Innenkanüle ermöglicht ihm zu sprechen. Die ungefensterte Innenkanüle bietet ihm dagegen Aspirationsschutz. Die Außenkanüle einer derartigen zweilumigen Sprechkanüle weist dabei immer ein Fenster auf, welches im Inneren der Trachea in der Regel nach oben hin (in Richtung Rachenraum) offen ist. Wahlweise kann dort zusätzlich noch ein Ventil oder eine einfache Klappe vorgesehen sein. Wenn die Innenkanüle an derselben Stelle ein Fenster hat, das mit der Außenkanüle in Überdeckung gebracht worden ist, so kann der Patient beispielsweise das patientenferne (proximale) Ende der Kanüle durch ein Ventil oder durch Druck mit dem Finger verschließen, so daß ausgeatmete Luft dann durch die sich überdeckenden Fenster und damit zum Kehlkopf gelangen kann, so daß der Patient sprechen kann. Da bei Verwendung der zweilumigen Kanülen der Austausch der Innenkanüle sehr einfach vonstatten geht und auch vom der Innenkanüle sehr einfach vonstatten geht und auch vom Patienten selbst vorgenommen werden kann, kann er also wahlweise Innenkanülen mit Fenstern durch Innenkanülen ohne Fenster ersetzen, wobei Innenkanülen ohne Fenster das Fenster der Außenkanüle weitgehend dicht verschließen, so daß Speisen, Flüssigkeiten oder sich ansammelnder Schleim nicht in das Innere der Kanüle eindringen können. Die gefensterte Innenkanüle wird also nur dann benutzt, wenn der Patient sprechen will. Die gefensterte Innenkanüle kann aber eventuell auch dauerhaft verwendet werden, wenn das Fenster der Außenkanüle ein Ventil aufweist, das nur Luft nach außen treten läßt.

Es versteht sich, daß das "Fenster" in der Außenkanüle und ebenso in der Innenkanüle nicht jeweils eine einzige, fensterartige Öffnung sein muß, sondern vielmehr auch aus einer Mehrzahl kleinerer und größerer Öffnungen bestehen kann, die zweckmäßigerweise jedoch auf einen gewissen Bereich beschränkt sind, der dann insgesamt als "Fenster" oder auch als "Fensterbereich" bezeichnet wird.

Allerdings können die anatomischen Verhältnisse im Halsbereich individuell sehr stark variieren. Bei frisch durchgeführten Tracheostomien sieht der Arzt in den meisten Fällen erst anhand von bronchoskopischen bzw. Röntgenuntersuchungen, inwieweit die verwendete Kanüle für den Patienten paßt. Da sich die richtige Länge nur sehr schwer im voraus feststellen läßt, kommt es häufig vor, daß die Fenestrierung von Sprechkanülen nicht frei in der Trachea liegt, sondern an die Wand der Trachea oder an das Gewebe angepreßt wird. In diesen Fällen kann die Luft nicht frei durch die Fenestrierung entweichen. D.h., der Patient kann nicht oder nur sehr schwer sprechen. In ungünstigen Fällen dringt die Luft durch die Fenestrierung in das Gewebe ein und es kommt zur Ausbildung von Halsemphysemen. Außerdem kann es geschehen, daß auch bei Verwendung einer Innenkanüle ohne Fenster oder wenn die Fenster von Außen und Innenkanüle einander nicht überlappen, durch einen kleinen, verbleibenden Spalt zwischen Außen- und Innenkanüle am distalen Ende Luft zwischen Außen- und Innenkanüle eindringt und über das möglicherweise nicht korrekt plazierte Fenster der Außenkanüle in das Umgebungsgewebe eindringt und ein Halsemphysem verursacht.

Neben dieser Problematik kann es aufgrund von ungeeigneten Kanülenlängen zu schwerwiegenden Problemen bei der Beatmung kommen.

Wenn der Arzt feststellt, daß die Länge der eingesetzten Kanüle nicht ideal paßt, bleibt ihm nichts anderes übrig, als sie auszutauschen. Dies ist stets mit gewissem Risiko behaftet. Sofern er weiterhin eine zweilumige Kanüle einsetzen möchte, hat er in der Regel nicht viele Alternativen, dem Patienten eine ideal passende Kanüle zur Verfügung zu stellen, da die Längenauswahl an zweilumigen Tracheostomiekanülen sehr beschränkt ist. Zwar ist es üblich, daß Hersteller von Tracheostomiekanülen ihre Fabrikate mit unterschiedlichen Außen- und Innendurchmessern anbieten. Bezüglich unterschiedlicher Längen gibt es jedoch nahezu keine Auswahl. Es gibt lediglich gewisse Unterschiede zwischen den verschiedenen Fabrikaten und Herstellern. Wenn man bedenkt, daß jeder Kanülenwechsel mit gewissen Risiken behaftet ist, so ist es kein befriedigender Zustand, wenn ein Arzt nach der "Trial and Error" Methode eine passende Kanüle suchen muß.

Das Problem wird dadurch verstärkt, daß sich der Halsdurchmesser (genauer der Abstand von Halsoberfläche zur Trachea) nach dem Eingriff aufgrund der verwendeten Therapie bzw. Wundheilung erheblich ändern kann. Das heißt, es kann zum Beispiel aufgrund von neu auftretenden oder sich rückbildenden. Gewebeschwellungen vorkommen, daß eine Kanüle, die ursprünglich ideal gepaßt hat, nach einiger Zeit nicht mehr paßt.

Gegenüber diesem Stand der Technik liegt der vorliegenden Erfindung die Aufgabe zugrunde, eine Tracheostomiekanüle mit Außenkanüle und Innenkanüle zu schaffen, mit welcher die Probleme unterschiedlicher Anatomien verschiedener Patienten oder auch die zeitliche Variation der Anatomie bei einem Patienten leicht behoben werden können.

Diese Aufgabe wird dadurch gelöst, daß die effektive Länge der Außenkanüle und/oder der Innenkanüle variabel ist.

Mit effektiver Länge (bezüglich der Außenkanüle) ist dabei in erster Linie die Länge des Abschnitts der Außenkanüle gemeint, der durch das Tracheostoma in die Trachea hineinragt und insbesondere der eventuell ein Fenster aufweisende Abschnitt. Der außerhalb des Körpers liegende Abschnitt der Außenkanüle wird in diesem Sinn nicht als Teil der "effektiven Länge" angesehen. Durch Veränderung der Länge des durch das Tracheostoma und in die Trachea hineinragenden Abschnitts der Tracheostomiekanüle kann diese den jeweiligen anatomischen Verhältnisses optimal angepaßt werden. Insbesondere kann die Länge so eingestellt werden, daß ein etwaiges Fenster an der Oberseite der Außenkanüle genau im Bereich der Trachea mündet. Bezüglich der Innenkanüle soll mit "effektiver Länge" in analoger Weise ebenfalls der Abschnitt beschrieben werden, der sich von dem Schild (der Außenkanüle) bis zum distalen Ende der Innenkanüle erstreckt. Ersatzweise könnte man die effektive Länge der Innenkanüle auch von der proximalen Öffnung der Außenkanüle bis zum distalen Ende der Innenkanüle messen. Eine Änderung der effektiven Länge der Innenkanüle bedeutet also, die Innenkanüle mehr oder weniger tief in die Außenkanüle einschieben zu können und in jeder dieser Positionen fixieren zu können.

Für diese Einstellung der variablen effektiven Länge der Tracheostomiekanüle gibt es verschiedene Möglichkeiten. Zum einen kann die Außenkanüle einen bei Bedarf dehnbaren Abschnitt aufweisen. Man wird dann in der Regel eine Außenkanüle bzw. Tracheostomiekanüle wählen, deren effektive Länge eher zu kurz bzw. grenzwertig kurz ist, wobei diese effektive Länge durch Dehnen des dehnbaren Abschnitts vergrößert wird, um auf diese Weise die Lage der Kanüle und insbesondere eines zum Sprechen vorgesehenen Fensters der Kanüle in der Trachea zu optimieren.

In einer anderen Ausführungsform ist das proximale Ende der Außenkanüle beispielsweise mit einem entlang der Kanüle verschiebbaren und in unterschiedlichen axialen Positionen fixierbaren Schild versehen. Ein solches Schild liegt im allgemeinen an dem das Tracheostoma umgebenden Bereich des Halses eines Patienten an und definiert damit auch die effektive Länge bzw. Position der Kanüle innerhalb der Trachea. Durch Verschieben des Schildes entlang eines proximalen Abschnitts der Tracheostomiekanüle verändert sich dann auch die effektive Länge der Kanüle, gemessen von dem Schild bzw. der Position des Schildes bis zum distalen inneren Ende Dabei werden die Begriffe "distal" und "proximal" aus der Sicht eines behandelnden Arztes verwendet, d.h. das proximale Ende (auch als "Maschinenende" bezeichnet) der Tracheostomiekanüle liegt außerhalb des Patientenkörpers und des Tracheostomas während das distale Ende (auch als "Patientenende" bezeichnet) im Inneren der Trachea liegt.

Bei der zuletzt erwähnten Ausführungsform ist es zweckmäßig, wenn das proximale Ende der Außenkanüle einen konstanten Außendurchmesser hat, obwohl eine Fixiervorrichtung des verschiebbaren Schildes vorzugsweise einen gewissen Spielraum für den Eingriff mit diesem proximalen Ende hat, insbesondere auch mit Außenkanülen, die einen geringfügig variierenden Außenumfang haben, in Eingriff treten kann. Entsprechende Schilde haben zum Beispiel eine Klemmvorrichtung, die die Außenkanüle von außen umfaßt und umklammert so daß das Schild dadurch an einer gewünschten Position fixiert wird. Derartige Klemmvorrichtungen sind im allgemeinen für einen gewissen Durchmesserbereich geeignet, so daß der Außendurchmesser des proximalen Endabschnittes, auf welchem das Schild fixiert werden soll, innerhalb eines solchen Durchmesserbereiches variieren darf, ohne die Klemmfunktion der Schildhalterung zu beeinträchtigen.

Besonders bevorzugt ist eine Ausführungsform der Erfindung, bei welcher die Innenkanüle gegenüber der Außenkanüle drehbar, d.h. um die gemeinsame Längsachse relativ zur Außenkanüle drehbar ist. Zwar verläuft diese Achse im allgemeinen leicht gekrümmt über einen Radius in der Größenordnung von einigen cm, wenn jedoch die Innenkanüle aus einem hinreichend flexiblen Material hergestellt wird, ist es dennoch möglich, sie um diese gekrümmte Achse zu drehen. Dies ermöglicht insbesondere die Verwendung von mit Fenster versehenen Innenkanülen, bei welchen die Innenkanüle zum einen so verdreht werden kann, daß ihr Fenster auf der dem Fenster der Außenkanüle gegenüberliegenden Seite angeordnet ist und somit beide Kanülenfenster geschlossen sind, während in einer anderen Drehposition, in welcher beispielsweise die beiden Fenster von Innenkanüle und Außenkanüle einander überlappen, ein Sprechen für den Patienten möglich ist, indem er Atemluft durch diese einander mindestens teilweise überdeckenden Fenster entweichen läßt.

In einer Ausführungsform ist die Innenwand der Außenkanüle von ihrem proximalen Ende zum distalen Ende hin konisch verjüngt ausgebildet. In eine anderen Ausführungsform ist die Innenwand der Innenkanüle von ihrem proximalen Ende zum distalen Ende hin öder in umgekehrter Richtung konisch verjüngt ausgebildet.

Zweckmäßig ist es weiterhin, wenn die Innenkanüle, konkret die Außenwand der Innenkanüle und die Innenwand der Außenkanüle leicht konisch ausgebildet sind, zum Beispiel in der Weise, daß der Innendurchmesser der Außenkanüle am distalen Ende um 1 mm kleiner ist als am proximalen Ende, wobei die Innenkanüle den entsprechenden Außendurchmesser hat. Aufgrund dieser leichten Konizität des Innenlumens der Außenkanüle kann die Innenkanüle zur proximalen Seite hin leichter aus der Außenkanüle entfernt werden.

In der Praxis werden jedoch häufig auch Außenkanülen mit (über ihre Länge hinweg) konstantem Durchmesser verwendet, wobei auch die Innenkanüle einen konstanten Durchmesser haben kann oder in umgekehrter Richtung, d.h. zumindest auf der Innenseite mit einer konischen Verjüngung zum proximalen Ende hin ausgebildet sein kann. Dies erleichtert für bestimmte Ausführungsformen die spritzgußtechnische Herstellung.

Besonders zweckmäßig ist es dabei, wenn der distale Endabschnitt des Lumens, d.h. der Innenwand,der Außenkanüle sich unabhängig von bzw. zusätzlich zu einer vorhandenen oder nicht vorhandenen, generellen, sehr geringen Gesamtkonizität unter einem größeren Konuswinkel verjüngt, der, gegenüber der Achse der Kanüle gemessen, mindestens 5° betragen sollte und beispielsweise zwischen 5° und 30°, vorzugsweise 10°-15° betragen kann. Dabei sollte das konisch verjüngte Ende der Außenkanüle so bemessen sein, daß sein kleinster Innendurchmesser etwas geringer ist als der Außendurchmesser am distalen Ende der Innenkanüle, damit die den kleinsten Innendurchmesser der Außenkanüle definierende, innere Abschlußkante der Außenkanüle in einen mehr oder weniger linienförmigen, dichten Eingriff mit der Außenwand der Innenkanüle treten kann. Hierdurch wird ein unbeabsichtigtes Eindringen von Atemluft in einen distalen Spalt zwischen Außen- und Innenkanüle sicher verhindert. Die Länge dieses sich konisch verjüngenden Endabschnittes sollte dabei vorzugsweise zum Beispiel zwischen 0,5 und 30 mm betragen und die Verjüngung über diesen Abschnitt ist vorzugsweise auf eine Durchmesserdifferenz von 0,1 bis 1,5 mm begrenzt. Es versteht sich weiterhin, daß die Wand dieses Endabschnittes nicht exakt konisch sein muß sondern im axialen Längsschnitt auch konvex oder konkav gewölbt oder stufenförmig verjüngt sein kann. Insbesondere kann eine Verjüngung am inneren distalen Ende der Außenkanüle auch durch einen ringförmig umlaufenden Wulst mit einem abgerundeten Querschnitt gebildet werden, der, ebenso wie die innere Kante einer konischen Verjüngung effektiv einen integrierten Dichtungsring für den linienförmigen Eingriff mit der Außenwand der Innenkanüle bildet.

Als Alternative zu der konischen Verjüngung des inneren Endabschnittes der Außenkanüle könnte auch eine separate Dichtung zwischen Außenkanüle und Innenkanüle in dem distalen Endabschnitt vorgesehen sein.

Wie bereits erwähnt, ist eine Ausführungsform der Erfindung besonders bevorzugt, bei welcher Innenkanüle und Außenkanüle je ein Fenster aufweisen, wobei die beiden Fenster bei vollständig eingesetzter Innenkanüle in axialer Richtung mindestens überlappen. Die Position der Fenster wird dabei abhängig von der Anatomie des Patienten variiert, d.h. die effektive Länge der Kanüle wird nach Bedarf verändert. Denkbar wäre es auch, unterschiedliche (Außen-)Kanülen vorzusehen, deren Fensterposition von Kanüle zu Kanüle variiert, so daß auf jeden Fall eine Kanüle für einen konkreten Patienten geeignet ist, indem das Fenster dieser in das Tracheostoma eingesetzten Kanüle im Bereich der Trachea des Patienten liegt.

In einer Ausführungsform machen die Fenster in Umfangsrichtung jeweils weniger als die Hälfte, vorzugsweise weniger als ein Drittel des Gesamtumfangs der jeweiligen Kanüle aus.

Die Fenster können unterschiedliche Größen und Formen haben und sie sollten in der bevorzugten Ausführungsform ein Breitenmaß (gemessen in Umfangsrichtung der Kanüle) von etwa 4 bis 8 mm und in axialer Richtung ein Mindestmaß von etwa 8 mm haben. Wenn das Fenster aus mehreren Öffnungen bzw. einer Gruppe kleinerer Öffnungen besteht, kann die Gesamtfläche, die von einer solchen Gruppe beansprucht wird, einschließlich der verbleibenden Zwischenräume auch deutlich größer sein, sollte jedoch vorzugsweise immer so bemessen, sein, daß der gesamte Fensterbereich ohne direkten Kontakt zu Körpergewebe in der Trachea frei liegen kann, wobei die Summe der Öffnungsquerschnitte der Gruppe von kleineren Öffnungen größer sein sollte als ein entsprechender Querschnitt einer einzelnen Fensteröffnung. Die von einem Fenster beanspruchte Fläche sollte 2 cm² möglichst nicht überschreiten. Besonders bevorzugt ist eine Ausführungsform der Erfindung, bei welcher das Fenster der Innenkanüle axial länger ausgebildet ist als das Fenster der Außenkanüle und beispielsweise eine um mindestens 50% größere axiale Länge hat. Dies ermöglicht es, bei Außenkanülen mit gleichem Innendurchmesser, jedoch unterschiedlichen Fensterpositionen, jeweils die gleiche Innenkanüle zu verwenden, wobei das Fenster der Innenkanüle trotz der unterschiedlichen Lagen der Fenster der Außenkanülen immer vollständig oder zumindest weitgehend mit dem Fenster der Außenkanüle überlappt und dieses letztlich dasjenige effektive Fenster definiert, das zur Trachea hin offen ist.

In einer Ausführungsform besteht mindestens eines der Fenster aus einer Mehrzahl von Öffnungen, die vorzugsweise auf einen Fensterbereich von maximal 2 cm² begrenzt sind.

Wird die Innenkanüle dann gegenüber der Position, in welcher die Fenster sich überdecken, um 180° um ihre Achse verdreht, so wird das Fenster der Außenkanüle durch die dem Fenster der Innenkanüle gegenüberliegende Wand der Innenkanüle verschlossen, und umgekehrt verschließt die dem Fenster der Außenkanüle gegenüberliegende Wand der Außenkanüle das Fenster der Innenkanüle.

Zweckmäßigerweise werden Innenkanülen und Außenkanülen so aufeinander abgestimmt, daß in allen praktisch vorkommenden relativen axialen Positionen zwischen Innenkanüle und Außenkanüle die - bei entsprechend gedrehter Innenkanüle - vorhandene Überlappungsfläche beider Fenster mindestens eine Fläche von 3 x 5 mm², oder alternativ mindestens 10% der freien Querschnittsfläche des kleineren der beiden überlappenden Fenster umfaßt.

Besonders bevorzugt ist eine Ausführungsform der Erfindung, bei welcher die Innenkanüle in unterschiedlichen axialen und Winkelpositionen relativ zur Außenkanüle einstellbar ist. Dies vergrößert nochmals den Verwendungsbereich ein und derselben Innenkanüle für verschiedene Außenkanülen, d.h. für Außenkanülen mit unterschiedlichen Fensterpositionen. Durch die axiale Verstellung der Innenkanüle wird die Lage des Fensters der Innenkanüle dann dem Fenster der Außenkanüle angepaßt. Besonders bevorzugt ist es, wenn diese unterschiedlichen axialen Positionen und Winkelpositionen der Innenkanüle in der Außenkanüle fixierbar sind, wobei es im allgemeinen ausreicht, wenn entsprechende Halterungselemente oder aber Außenkanüle und Innenkanüle als Ganzes in ausreichendem Reibeingriff miteinander stehen, so daß sich die Position der Innenkanüle relativ zur Außenkanüle nicht willkürlich bzw. selbsttätig verändert.

In einer Ausführungsform ist beispielsweise vorgesehen, daß Außenkanüle und Innenkanüle über ein Gewinde miteinander verbunden sind, so daß es von der Anzahl der relativen Drehungen der Innenkanüle relativ zu der Außenkanüle, wenn diese in Gewindeeingriff miteinander stehen, abhängt, welche axiale Position die Innenkanüle einnimmt, wobei bei einer gewünschten axialen Position, in welcher die Fenster von Außenkanüle und Innenkanüle eine maximale Überlappung haben, noch immer eine relative drehung der Innenkanüle gegenüber der Außenkanüle um ± 180° möglich ist, um die beiden Fenster zu verschließen. Alternativ kann die axiale Position der Innenkanüle gegenüber der Außenkanüle mit Hilfe einer Überwurfmutter eingestellt und fixiert werden.

Die axiale Verstellbarkeit der Innenkanüle kann man auch dafür verwenden, die Sprachfunktion, konkret die Überlappung der Fenster von Innen- und Außenkanüle ein- und auszuschalten. Wenn die axiale Position der Innenkanüle an der Außenkanüle beispielsweise mit Hilfe eines Gewindes eingestellt wird, können die Fenster von Innen- und Außenkanüle so ausgestaltet werden, das de Überlappung der Fenster innerhalb des axialen Einstellbereiches liegt. D.h. in einer einstellbaren axialen Position der Innenkanüle relativ zur Außenkanüle überlappen die beiden Fensterbereiche nicht, während in einer anderen einstellbaren Axialposition die Überlappung der Fenster ausreicht, um die zum Sprechen erforderliche Atemluft durch die Fenster in Richtung des Rachenraumes abzuleiten.

Eine weitere, bevorzugte Ausführungsform ist dadurch gekennzeichnet, daß mindestens die Außenkanüle einen ovalen oder ovalisierbaren distalen Endabschnitt aufweist, wobei die Kanüle auch über ihre gesamte Länge hinweg einen solchen Querschnitt haben kann, der in horizontaler Richtung, d.h, quer zu den Trachealspangen ein größeres und senkrecht zu den Trachealspangen ein kleineres Maß hat. Dies vermindert die Gefahr eines Bruches der Trachealspangen beim Dilatieren und/oder beim Einbringen der Außenkanüle in das Tracheostoma, wobei die Kanüle nach dem Einsetzen auch ohne weiteres wieder einen kreisrunden Querschnitt annehmen kann

Im Folgenden werden noch einige Ausführungsformen und Varianten der erfindungsgemäßen Tracheostomiekanüle beschrieben.

Beispielsweise könnte am distalen Ende der Außenkanüle ein Anschlag für das distale Ende der Innenkanüle vorgesehen sein. Dieser Anschlag sollte unabhängig und zusätzlich zu einer etwaigen konischen Verjüngung bzw. unabhängig von einer umlaufenden zylindrischen Dichtung, wie sie beispielsweise durch den minimalen Durchmesser bzw. die Kante des Dichtungskonus gebildet wird, vorgesehen sein. Darüber hinaus könnte bei einer solchen Ausführungsform am proximalen Ende der Innenkanüle ein Flansch vorgesehen sein und entsprechend könnte man an der Außenkanüle ein Gewinde oder eine Art Bajonetteingriff vorsehen mit einer Überwurfmutter, die in das Gewinde eingreift bzw. eine entsprechende Bajonettfassung bzw. ein Bajonettgegenstück bildet. Zweckmäßigerweise ist mit Hilfe einer solchen Überwurfmutter die axiale Position der Innenkanüle relativ zur Außenkanüle einstellbar, indem die Überwurfmutter an dem Flansch der Innenkanüle angreift und diese mehr oder weniger tief in die Außenkanüle hineinschiebt. Wenn dabei das distale Ende der Innenkanüle mit dem Anschlag am distalen Ende der Außenkanüle in Eingriff tritt, wird eine Dehnungskraft zwischen dem proximalen und distalen Ende der Außenkanüle ausgeübt, so daß ein Dehnungsabschnitt der Außenkanüle, der vorzugsweise im Bereich zwischen einem Fenster der Kanüle und dem proximalen Ende liegt, ausgeübt und über diese Dehnung kann dann die Lage des Fensters genauer eingestellt und fixiert werden. Wenn der Dehnungsabschnitt der Außenkanüle elastisch dehnbar ist, ist diese Einstellung reversibel. Der betreffende Abschnitt kann aber auch inelastisch dehnbar sein, so daß eine einmal vorgenommene Dehnung nicht oder nur in geringem Maße wieder rückgängig zu machen ist.

Anstelle eines Fensters, welches in der Regel auf der zum Rachenraum hingewandten Seite der Außenkanüle vorgesehen ist, könnte auf der gegenüberliegenden Seite ein weiteres Fenster (welches wiederum aus einer Gruppe kleinerer Fensteröffnungen bestehen kann) vorgesehen sein. Im allgemeinen ist die Trachea deutlich weiter als es dem Durchmesser der Außenkanüle entspricht, so daß bei Verwendung einer solchen Kanüle mit Fenstern auf beiden Seiten ein Patient auch dann sprechen kann, wenn beispielsweise das obere Fenster an der Wand der Trachea anliegt, was dann nahezu zwangsweise zur Folge hat, daß das gegenüberliegende Fenster frei ist, wobei die Luft zum Sprechen auch durch das untere Fenster und an der Kanüle vorbei nach oben über den Kehlkopf zum Rachenraum hin ausströmen kann.

Die Außenkanüle muß auch nicht zwingend einen Cuff aufweisen. Ein Cuff wird insbesondere benötigt bei der mechanischen Beatmung von Patienten, um zu verhindern, daß die durch die Kanüle in die Trachea gepumpte Luft aufgrund des Widerstandes in den Bronchien und der Lunge, sondern an der Außenseite der Kanüle zurück in den Rachenraum strömt und dadurch nicht genug Sauerstoff in die Lunge gelangt. In vielen Fällen, insbesondere wenn der Patient aktiv atmet oder die maschinelle Beatmung unterstützt oder auch im Falle einer sehr engen Trachea wie z.B. bei Kindern ist jedoch mitunter kein Cuff erforderlich. Der Cuff soll außerdem verhindern, daß Sekret und sonstige Flüssigkeiten, die sich möglicherweise oberhalb des Cuffs in der Trachea ansammeln können, in die Bronchien bzw. die Lunge des Patienten gelangen können.

Bei einer besonders bevorzugten Ausführungsform der Erfindung ist zusätzlich zu der Außenkanüle und der Innenkanüle eine dritte Zwischenkanüle vorgesehen, die nur die Funktion eines Schiebers hat, die zwischen der Außenkanüle und der Innenkanüle angeordnet ist, und welche so ausgebildet ist, daß sie die Verbindung zwischen dem Fenster der Innenkanüle und dem damit ausgerichteten Fenster der Außenkanüle wahlweise blockiert oder freigibt. Bei dieser Ausführungsform sind die Fenster von Außenkanüle und Innenkanüle dauerhaft miteinander ausgerichtet und die dazwischenliegende Zwischenkanüle ist entweder axial beweglich oder drehbeweglich, und kann wahlweise den Durchgang zwischen dem Fenster der Innenkanüle und dem der Außenkanüle freigeben oder blockieren. Beispielsweise könnte die Zwischenkanüle eine drehbare Hülse sein, welche auf zwei gegenüberliegenden Seiten zwei Fenster hat, die in etwa die axiale Lage und auch die Größe der Fenster von Außen- und Innenkanüle haben, so daß in einer Orientierung mindestens eines der Fenster dieser Hülsen mit den Fenstern von Außen- und Innenkanüle ausgerichtet ist, so daß Luft durch die Fensteröffnungen hindurchströmen kann, während in einer um 90° verdrehten Position die zwischen den Fensteröffnungen liegenden Wandabschnitte der Zwischenhülse den Durchgang zwischen dem Fenster der Innenkanüle und dem der Außenkanüle blockieren. Eine solche Zwischenhülse muß sich nicht über die volle Länge von Außenkanüle und Innenkanüle erstrecken, sondern es würde vielmehr ausreichen, wenn eine solche Zwischenkanüle bis über die Position der Fenster der Außen- oder Innenkanüle hinausragt. Eine solche Zwischenkanüle läßt sich, da sie über einen entsprechend kurzen Abschnitt weniger stark gekrümmt ist, leichter um ihre Achse drehen als eine Innenkanüle, die sich in gekrümmter bzw. gebogener Form über die gesamte Länge der Außenkanüle erstreckt.

In einer weiteren bevorzugten Ausführungsform der Erfindung weist das Schild, welches durch Anlage am Hals des Patienten die Position der Außenkanüle in der Trachea festlegt, auf seiner dem Patienten zugewandten Seite ein fluidgefülltes Kissen auf, das beispielsweise getrennt aufblasbar und auch wieder entlüftbar ist. Ein solches Kissen kann den Tragekomfort erhöhen, wobei durch Änderung der in dem Kissen enthaltenen Menge an Fluid, d.h. durch weiteres Aufpumpen und/oder Entleeren zusätzlich auch die Position des Schildes bezüglich des Halses des Patienten verändert und auch dauerhaft eingestellt werden kann, um auf diese Weise die Position der Kanüle in der Trachea zu beeinflussen, beispielsweise, um das Fenster der Außenkanüle in eine gewünschte, korrekte Lage in der Trachea zu bringen.

Weitere Vorteile, Merkmale und Anwendungsmöglichkeiten der vorliegenden Erfindung werden deutlich anhand der folgenden Beschreibung einer bevorzugten Ausführungsform und der dazugehörigen Figuren. Es zeigen:
- Figur 1: schematisch eine erfindungsgemäße Außenkanüle mit Schild, Cuff und Kontrollballon
- Figur 2: eine entsprechende Innenkanüle mit einem Außengewinde an ihrem proximalen Ende, und
- Figur 3: Außen- und Innenkanüle in zusammengesetztem Zustand
- Figur 4: eine Außenkanüle mit zwei gegenüberliegend angeordneten Fenstern
- Figuren 5 und 6: eine Ausführungsform einer Außenkanüle mit ihrem distalen Ende im Längsschnitt und mit einer eingesetzten Innenkanüle in zwei verschiedenen axialen Positionen.
- Figur 7: den distalen Endabschnitt einer Außenkanüle mit eingesetzter Innenkanüle in einer alternativen Ausführungsform

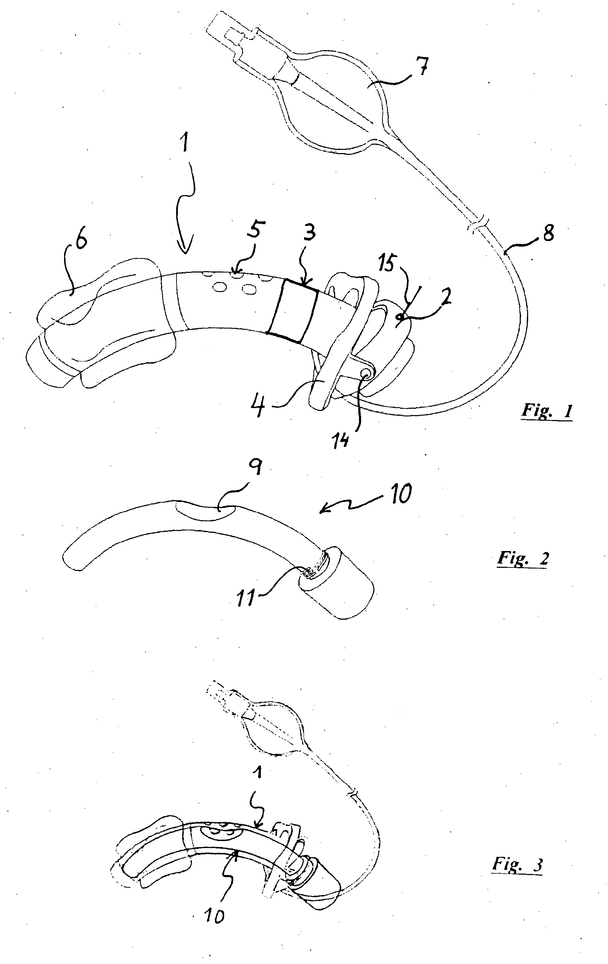

In Figur 1 erkennt man die insgesamt mit 1 bezeichnete Außenkanüle in Form eines gekrümmten Rohres, welches an seinem freien proximalen Ende ein verschiebbares und vorzugsweise um zwei Achsen schwenkbares Schild 4 aufweist, welches zur Anlage an die ein Tracheostoma umgebende Halsoberfläche eines Patienten vorgesehen ist. An dem distalen Ende der Tracheostomiekanüle 1 erkennt man einen Cuff 6, der über einen Ballon 7 und einen durch die Wand oder außen auf der Wand verlaufenden Füllschlauch 8 auf einen leichten Überdruck von typischerweise etwa 20 mbar aufpumpbar ist und sich dabei an die Innenwand der Trachea des Patienten anlegt, nachdem die Tracheostomiekanüle 1 durch ein entsprechendes Tracheostoma in die Trachea eingeführt worden ist.

Die Außenkanüle 1 weist außerdem noch ein Fenster 5 auf, welches durch mehrere kleine Öffnungen gebildet wird, wobei die Summe der Öffnungsquerschnitte der einzelnen Öffnungen des Fensters 5 größer sein sollte als der Innenquerschnitt der Außenkanüle. Alternativ könnte, wie auch bei der Innenkanüle in Figur 2 erkennbar, eine einzelne, ovale Fensteröffnung vorgesehen sein, deren Maß in Umfangsrichtung typischerweise etwa 5 bis 6 mm und in axialer Richtung typischerweise etwa 10 bis 15 mm beträgt, wobei selbstverständlich auch andere Maße und insbesondere auch andere Formen solcher Fenster möglich sind. Das Fenster 5 könnte auch eine Ventilklappe oder einen Ventilschlauch oder dergleichen aufweisen, die lediglich ein Ausströmen von Luft aus der Tracheostomiekanüle 1 durch das Fenster 5 in die Trachea ermöglichen, jedoch keine Strömung in umgekehrter Richtung zulassen bzw. diese Strömung in umgekehrter Richtung zumindest stark behindern oder dämpfen.

Ein umlaufender Abschnitt 3 der Tracheostomiekanüle 1, der hier in Form eines mit stärkeren Linien umrissenen, schwarzen Rechtecks dargestellt ist, ist ein dehnbarer Bereich, dessen Länge beispielsweise bis auf das Doppelte der dargestellten axialen Länge ausgedehnt werden kann. Außerdem ist in der bevorzugten Ausführungsform der Erfindung das Schild 4 verschiebbar auf dem proximalen Ende der Tracheostomiekanüle 1 befestigt. Beides ermöglicht es, zumindest das distale Ende der Tracheostomiekanüle 1, welches das Fenster 5 aufweist, in der Trachea und am Übergang von der Trachea in das Tracheostoma so weit zu verschieben, daß das Fenster vollständig innerhalb des Bereichs der Trachea, und zwar kurz vor dem Tracheostoma liegt, so daß das Fenster 5 im wesentlichen keine Wandberührung hat, sondern in den nach oben offenen Bereich der Trachea mündet. Beim Einsetzen der Tracheostomiekanüle 1 kann diese Lage des Fensters 5 mit Hilfe eines Bronchoskops oder dergleichen kontrolliert und überwacht werden, wobei gegebenenfalls der Bereich 3 etwas gedehnt und/oder das Schild 4 in axialer Richtung etwas verschoben und neu fixiert wird.

Auch wenn der dehnbare Bereich 3 je nach Ausführungsform möglicherweise nur ein einziges Mal (inelastisch) gedehnt werden kann, bietet das verschiebbare Schild 4 immer noch die Möglichkeit, etwaige anatomische Veränderungen, die zum Beispiel im Verlauf eines Heilungsprozesses des Tracheostomas auftreten können, zu kompensieren, so daß das Fenster 5 seine optimale Lage behält.

Das Schild 4 ist um eine Achse 14 verschwenkbar an einem Ring 2 befestigt, welcher seinerseits um eine zu der Achse 14 senkrechte Achse 15 bezüglich der Außenkanüle 1 verschwenkbar ist. In einer bevorzugten Ausführungsform ist der Ring 2 in axialer Richtung, vorzugsweise gegen elastische Rückstellkräfte, verschiebbar, Beispielsweise sind zwei die Achse 15 definierende und sich von gegenüberliegenden Innenseiten des Ringes 2 einwärts erstreckende Achsstutzen (hier nicht erkennbar) in zwei Nuten oder Schienen am proximalen Endabschnitt der Außenkanüle 1 geführt. Vorzugsweise ist der Ring 2 bzw. sind dessen Achsstutzen in dieser Schiene elastisch gehalten, so daß der Ring sich unter Überwindung elastischer Rückstellkräfte in axialer Richtung der Außenkanüle 1 um einige Millimeter vor- oder zurückbewegen kann. Insgesamt sollte das Bewegungsspiel des Ringes 2 in einer bevorzugten Ausführungsform +/- 5 mm betragen.

In Figur 2 erkennt man eine entsprechende Innenkanüle 10, deren Außendurchmesser auf den Innendurchmesser der Außenkanüle 1 abgestimmt ist und die dementsprechend in die Außenkanüle eingesetzt werden kann. Die Innenkanüle 10 ist zwar weitgehend formstabil, insbesondere was ihren Öffnungsquerschnitt bzw. das innere Lumen angeht, ist jedoch ansonsten vorzugsweise flexibel und gut biegsam. Dies bedeutet, daß die Innenkanüle auch in ihrem in die Außenkanüle eingesetzten Zustand relativ zu der Außenkanüle 1, die demgegenüber vergleichsweise steif ist, um ihre Längsachse gedreht werden kann, so daß auch die Position des Fensters 9 in der Innenkanüle in Umfangsrichtung relativ zu dem Fenster 5 der Außenkanüle einstellbar ist. Außerdem hat das Fenster 9 der Innenkanüle eine größere axiale Länge als das Fenster 5 bzw. die Gruppe 5 der Fensteröffnungen der Außenkanüle, was im Ergebnis dazu führt, daß ein und dieselbe Innenkanüle 10 für verschiedene Außenkanülen verwendbar ist oder auch für ein und dieselbe Außenkanüle verwendbar ist, unabhängig davon, ob die Außenkanüle in dem Dehnungsbereich um einige Millimeter, beispielsweise um 5 bis 10 mm, gedehnt wurde.

Ohne daß dies in den Zeichnungen erkennbar ist, sind Außenkanüle 1 und Innenkanüle 10 leicht konisch ausgebildet, d.h. konkret verläuft die Innenwand der Außenkanüle bei diesem Ausführungsbeispiel vom proximalen zum distalen Ende hin leicht konisch verjüngt und entsprechend verjüngt sich auch der Außendurchmesser der Innenkanüle vom proximalen zum distalen Ende hin oder, im Falle der Innenkanüle, aus spritzgußtechnischen Gründen umgekehrt. Diese Durchmesseränderung beträgt aber über die Länge der gesamten Innen- bzw. Außenkanüle im allgemeinen nicht mehr als maximal 1 mm, so daß die Innenkanüle auch in Ihrem vollständig in die Außenkanüle eingesetzten Zustand noch ein gewisses axiales Spiel von zum Beispiel 5 mm in beiden Richtungen hat. Wie bereits erwähnt, könnten eine oder beide Kanülen über ihre Länge hinweg auch einen konstanten Durchmesser bzw. Querschnitt haben. Erkennbar ist allerdings eine zusätzlich, deutlich konische Ausbildung des distalen inneren Endabschnittes der Außenkanüle auf einen minimalen Durchmesser, der geringfügig kleiner ist als der Außendurchmesser des distalen Endes der Innenkanüle, das dadurch mit der innere Endkante des konisch verjüngten Endabschnittes der Außenkanüle in dichtenden Eingriff treten kann, wenn die Innenkanüle in diesen Endabschnitt der Außenkanüle hineingeschoben bzw. hindurch geschoben wird. Eventuell wird eine dichtender Eingriff auch bereits dann erricht, wenn die vordere äußere Kante der Innenkanüle mit der konische Innenfläche des Endabschnittes der Außenkanüle in Kontakt tritt, ohne daß die Innenkanüle den Bereich des kleinsten Durchmessers (die innere Endkante) dieses konischen Endabschnittes erreicht.

Bei der hier dargestellten Ausführungsform hat die Innenkanüle an ihrem proximalen Ende ein Außengewinde 11, welches mit einem entsprechenden, in den Figuren nicht erkennbaren, Innengewinde am proximalen Ende der Außenkanüle 1 in Eingriff tritt und dementsprechend aufgrund der Anzahl entsprechender Umdrehungen der Innenkanüle 10 gegenüber der Außenkanüle 1 die Veränderung der axialen Position der Innenkanüle 10 relativ zur Außenkanüle 1 ermöglicht. Dabei kann die Innenkanüle, wenn die beiden Fenster 5, 9 zur Überdeckung gebracht worden sind, noch immer um weitere 180° vor- oder zurückgedreht werden, um das Fenster 9 auf die dem Fenster 5 gegenüberliegende Seite der Achse zu bringen und damit beide Fenster zu verschließen, wobei die damit verbundene geringe axiale Verschiebung der Innenkanüle zur Außenkanüle um die halbe Gewindeganghöhe ohne Bedeutung ist. Alternativ kann die Innenkanüle proximal einen Endflansch aufweisen, der durch eine Überwurfmutter, die in einen Gewindeabschnitt der Außenkanüle eingreift, axial fixierbar bzw. einstellbar ist,

In Figur 3 sind die Außenkanüle 1 und die Innenkanüle 10 in einem zusammengesetzten Zustand erkennbar. Wenn die beiden Fenster zur Überdeckung gebracht sind, kann der Patient mit Hilfe der durch die Fenster ausgestoßenen Atemluft sprechen, wenn das proximale Ende der Kanülen 1, 10 entweder durch ein Ventil oder durch Auflage eines Fingers oder sonstigen Stopfens verschlossen wird.

Wesentlich für die vorliegende Erfindung ist demnach, daß, um die erforderliche Anzahl verschiedener Kanülen so gering wie möglich zu halten und dennoch eine optimale Anpassung an unterschiedliche Verhältnisse bei Patienten vornehmen zu können, die effektive Länge der Außenkanüle, d.h. das Maß zwischen Schild und distalem Ende oder, auf bevorzugte Ausführungsformen bezogen, das Maß zwischen Schild und Position eines Fensters 5, variabel ist. Für die Anpassung der Innenkanüle gibt es unterschiedliche Möglichkeiten, wie zum Beispiel die axiale Verstellung der Innenkanüle innerhalb der Außenkanüle und die Vergrößerung des Fensters der Innenkanüle gegenüber dem Fenster der Außenkanüle, so daß ein und dieselbe Innenkanüle für unterschiedliche Außenkanülen oder auch für in ihrer Länge variabel eingestellte Außenkanülen verwendet werden kann.

Zweckmäßigerweise ist die Länge der Innenkanüle 10 geringfügig größer als die Länge der Außenkanüle, so daß, wie auch immer die Innenkanüle mit ihrem Fenster 9 relativ zu dem Fenster 5 eingestellt wird, das distale Ende der Innenkanüle ein kleines Stück weit aus dem distalen Ende der Außenkanüle herausragt oder zumindest bündig mit diesem abschließt. Dies stellt einen dichten Eingriff zwischen Innen- und Außenkanüle an deren verjüngten Ende sicher. Außerdem vermeidet man dadurch irgendwelche Verkrustungen oder Anbackungen auf der Innenseite des distalen Endes der Außenkanüle, wohingegen jegliche Verkrustungen oder Anbackungen an der Innenfläche der Innenkanüle leicht entfernt werden können, indem die Innenkanüle aus der Außenkanüle herausgezogen und extrakorporal gereinigt wird. Dabei kann die Innenkanüle auch kurzzeitig durch eine weitere Innenkanüle (mit oder ohne Fenster) ersetzt werden.

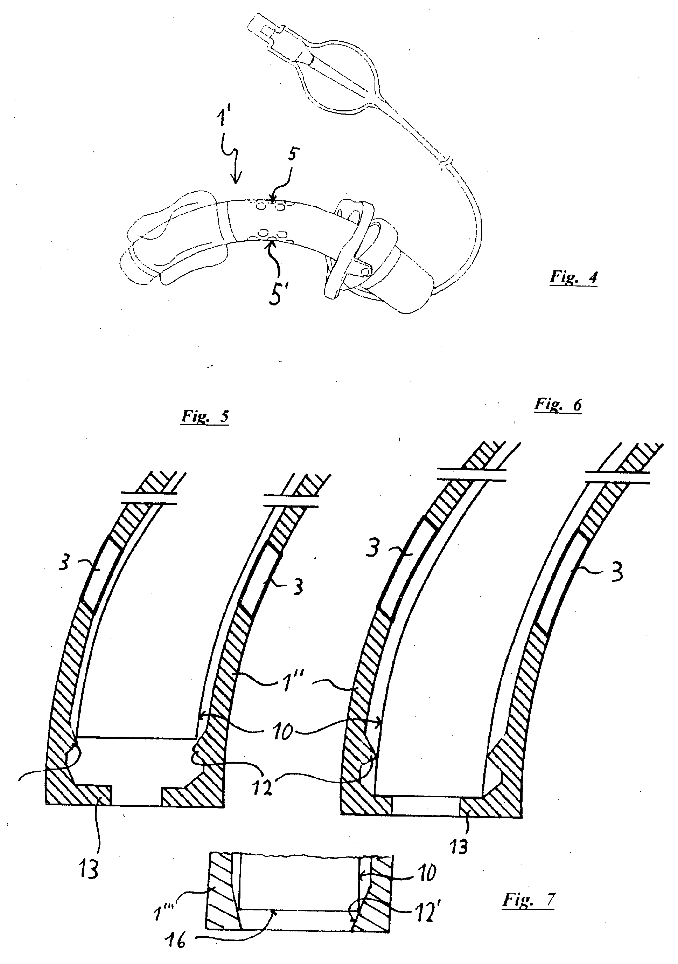

In Figur 4 erkennt man eine weitere Ausführungsform einer Außenkanüle 1', welche neben einem Fenster 5 auf der konvexen Seite des von der Außenkanüle 1' gebildeten Bogens auch auf der konkaven Seite dieses Bogens ein Fenster 5' aufweist, wobei die Fenster 5, 5' in beiden Fällen aus mehreren kleineren Fensteröffnungen bestehen.

Dies stellt sicher, daß selbst dann, wenn beispielsweise die Öffnungen des Fensters 5 vollständig oder fast vollständig an der Wand der Trachea anliegen, die Fensteröffnungen des gegenüberliegenden Fensters 5' mit hoher Wahrscheinlichkeit freiliegen, so daß Atemluft zum Sprechen aus den Öffnungen des Fensters 5' ausströmen und durch die Trachea nach oben an der Außenkanüle 1' vorbei über den Kehlkopf zum Rachenraum strömen kann, so daß der Patient, der eine solche Kanüle trägt, sprechen kann. Ebenso könnten die Fenster aber auch um nur 90° oder einen anderen Winkel in Umfangsrichtung versetzt sein, um die Wahrscheinlichkeit zu erhöhen, daß mindestens eines dieser Fenster in der Trachea freiliegt. Die Figuren 5 und 6 zeigen weitere Details bevorzugter Ausführungsformen der vorliegenden Erfindung. In den Figuren 5 und 6 sind Abschnitte einer Kanüle 1" in unterschiedlichen Zuständen dargestellt. Weiterhin erkennt man eine Innenkanüle 10, die in Figur 5 teilweise und in Figur 6 vollständig in die entsprechende Außenkanüle 1" eingeführt ist. Im Abstand von dem unteren, distalen Ende der Außenkanüle 1" erkennt man die bereits in Figur 1 beschriebenen, dehnbaren Bereiche 3, die hier zwar in der Nähe des distalen Endabschnittes dargestellt sind, im Prinzip jedoch an einer beliebigen Stelle der Außenkanüle auch nahe am proximalen Endabschnitt (jedoch distal bezüglich des das Schild 4 tragenden Ringes 2) angeordnet sein könnten. In diesem Fall weist die Außenkanüle 1" keinen Cuff auf, der aber, ebenso wie in Figur 1 dargestellt, auch trotz eines dehnbaren Bereiches 3 vorhanden sein könnte.

In Figur 5 erkennt man außerdem einen konisch verjüngten Dichtungsabschnitt 12 auf der Innenseite der Außenkanüle 1" kurz vor dem distalen Ende der Außenkanüle 1". Des weiteren ist das distale Ende der Außenkanüle 1 mit einem radial einwärts ragenden Flansch 13 versehen, der hier allerdings übertrieben groß dargestellt ist und der auch durch einzelne, radial einwärts ragende Stege oder auch sich kreuzende Stege ersetzt werden könnte. Wird die Innenkanüle 10' vollständig in die Außenkanüle 1" eingeschoben, so gleiten die vorderen Kanten des distalen Endes der Innenkanüle 10 auf die oberen Flächen des Dichtungskonus 12 auf, wobei die elastische Spitze bzw. Kante dieses umlaufenden Dichtungskonus radial nach außen gedrängt wird und schließlich mit der Außenwand des Innenkonus 10 in dichtendem Eingriff steht. Dabei entsteht eine linienförmig umlaufende Dichtung mit relativ großem spezifischem Flächendruck. Wenn das distale Ende der Innenkanüle 10 an den Endflansch 13 der Außenkanüle 1" anschlägt, kann durch fortgesetztes Hineinschieben der Innenkanüle 10 in die Außenkanüle 1" unter Festhalten des proximalen Endes der Außenkanüle 1" der dehnbare Abschnitt 3 gedehnt werden, wie man dies anhand der unterschiedlichen axialen Längen der Abschnitte 3 in den Figuren 5 und 6 erkennt. Dies ermöglicht eine Anpassung und Einstellung der Länge der Außenkanüle an gegebene anatomische Strukturen, so daß ein Fenster 5 bzw. 5' der Außenkanüle innerhalb einer Trachea korrekt plaziert werden kann. Es versteht sich, daß zu diesem Zweck die Innenkanüle 10 ausreichend lang sein muß und nach Möglichkeit an dem proximalen Ende der Außenkanüle 1" in unterschiedlichen axialen Positionen fixierbar sein muß. Hierzu dient beispielsweise die schon angesprochene Überwurfmutter, die das proximale Ende oder einen Flansch am proximalen Ende der Innenkanüle 10 erfaßt und auf ein Gewinde am proximalen Ende der Innenkanüle 1" aufgeschraubt wird.

Selbstverständlich ist es auch möglich, eine möglicherweise zu kurze Innenkanüle 10 durch eine längere Innenkanüle zu ersetzen, wenn der dehnbare Bereich 3 mit einer gegebenen Innenkanüle 10 nicht ausreichend gedehnt werden kann.

Figur 7 zeigt im übrigen noch eine alternative Ausgestaltung des distalen Endes einer Außenkanüle 1", die in diesem Fall keinen Endflansch aufweist, sondern lediglich einen Dichtungskonus 12'. Dabei kann der Dichtungskonus 12' so bemessen sein, daß beispielsweise die untere distale Kante 16 in dichtenden Eingriff mit einer Konusfläche tritt, wobei es aber ebenso gut denkbar wäre, daß die Innenkanüle 10' vollständig in die Außenkanüle 1'" eingeschoben wird, so daß sie bündig mit deren distalem Ende abschließt oder sogar ein Stück über das distale Ende der Außenkanüle hinausragt und auf diese Weise die Innenkante des Dichtungskonus 12 mit dem kleinsten Durchmesser mit der Außenfläche der Innenkanüle 10 in Eingriff setzt, um in ähnlicher Weise eine Abdichtung zu bewirken wie dies im Beispiel der Figur 6 an dem Innenkonus 12 dargestellt ist.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Dies gilt entsprechend auch für jeweils mehrere in einem abhängigen Anspruch aufgezählten Merkmale, die jeweils individuell verwirklicht werden können, sofern sie sich nicht unmittelbar aus technischen Gründen bedingen. Auf die umfassende, explizite Darstellung sämtlicher denkbarer Merkmalskombinationen wird hier nur der Kürze und der Lesbarkeit der Beschreibung wegen verzichtet.

## Patentansprüche

1. Tracheostomiekanüle, bestehend aus einer Außenkanüle (1) und einer darin passend einsetzbaren Innenkanüle (10), **dadurch gekennzeichnet, daß** die effektive Länge der Außenkanüle (1) und der Innenkanüle (10) variabel ist, wobei die Außenkanüle (1) und die Innenkanüle (10) mindestens je ein Fenster (5, 9) aufweisen, wobei die Fenster (5, 9) bei in die Außenkanüle (1) passend eingeschobener Innenkanüle (10) in axialer Richtung mindestens teilweise überlappen, wobei die Außenkanüle ein proximales Ende mit einem verschiebbaren Schild aufweist.

2. Tracheostomiekanüle nach Anspruch 1, **dadurch gekennzeichnet, daß** die Außenkanüle einen wahlweise dehnbaren Abschnitt aufweist.

3. Tracheostomiekanüle nach einem der Ansprüche 1 bis 2, **dadurch gekennzeichnet, daß** die Innenkanüle (10) gegenüber der Außenkanüle (1) drehbar ist.

4. Tracheostomiekanüle nach einem der vorstehenden Ansprüche, **dadurch gekennzeichnet, daß** das Fenster der Innenkanüle axial länger als das Fenster der Außenkanüle ausgebildet ist.

5. Tracheostomiekanüle nach einem der vorstehenden Ansprüche, **dadurch gekennzeichnet, daß** die Innenkanüle (10) in unterschiedlichen axialen und Winkelpositionen relativ zur Außenkanüle einstellbar ist.

6. Tracheostomiekanüle nach einem der vorstehenden Ansprüche, **dadurch gekennzeichnet, daß** die Innenkanüle in unterschiedlichen axialen und unterschiedlichen Winkelpositionen relativ zur Außenkanüle fixierbar ist.

7. Tracheostomiekanüle nach einem der vorstehenden Ansprüche, **dadurch gekennzeichnet, daß** die Innenkanüle in axialer Richtung und in Umfangsrichtung über ein Gewinde in der Außenkanüle einstellbar ist.

8. Tracheostomiekanüle nach Anspruch 6, **dadurch gekennzeichnet, daß** ein Außengewinde am proximalen Ende der Außenkanüle vorgesehen ist während das proximale Ende der Innenkanüle einen Flansch aufweist, der durch eine Überwurfmutter, die mit dem Außengewinde in Eingriff steht, an der Außenkanüle festgehalten wird.

9. Tracheostomiekanüle nach Anspruch 6, **dadurch gekennzeichnet, daß** je ein Außen- bzw.
ein Innengewinde am proximalen Ende von Innenkanüle und Außenkanüle vorgesehen ist, die miteinander in Eingriff bringbar sind.

10. Tracheostomiekanüle nach einem der vorstehenden Ansprüche, **dadurch gekennzeichnet, daß** am distalen Ende der Außenkanüle ein Anschlag für das distale Ende der Innenkanüle vorgesehen ist.

11. Tracheostomiekanüle nach einem der vorstehenden Ansprüche, **dadurch gekennzeichnet, daß** die Innenkanüle proximal einen Flansch bzw. ein Ende aufweist, welches von einer Überwurfmutter erfaßt und am proximalen Ende der Außenkanüle eingestellt und fixiert wird.

12. Tracheostomiekanüle nach einem der vorstehenden Ansprüche, **dadurch gekennzeichnet, daß** die Außenkanüle mindestens zwei Fenster an verschiedenen Umfangspositionen der Kanüle aufweist.

13. Tracheostomiekanüle nach einem der vorstehenden Ansprüche, **dadurch gekennzeichnet, daß** an der Außenkanüle und an der Innenkanüle vorgesehene Fenster derart ausgestaltet sind und die Axialposition der Innenkanüle im Verhältnis zur Außenkanüle derart variabel einstellbar ist, daß in einer ersten Axialposition die Fenster von Innen- und Außenkanüle nicht überlappen und in einer anderen soweit überlappen, daß durch die überlappenden Fenster ausreichend Luft zum Sprechen entweichen kann.

## Claims

1. Tracheostomy cannula consisting of an outer cannula (1) and an inner cannula (10), which can be inserted fittingly into the outer cannula, **characterized in that** the effective length of outer cannula (1) and inner cannula (10) is variable, wherein the outer cannula (1) and the inner cannula (10) each comprises at least one window (5, 9), wherein the windows (5, 9) when the inner cannula (10) is fittingly inserted into the outer cannula (1) overlap at least partially in the axial direction, wherein the outer cannula has a proximal end, having a movable neck flange.

2. Tracheostomy cannula according to claim 1, **characterized in that** the outer cannula optionally comprises a expendable portion.

3. Tracheostomy cannula according to one of claims 1 to 2, **characterized in that** the inner cannula (10) is rotatable relative to the outer cannula (1).

4. Tracheostomy cannula according to one of the preceding claims, **characterized in that** the window of the inner cannula is designed to be axially longer than the window of the outer cannula.

5. Tracheostomy cannula according to one of the preceding claims, **characterized in that** the inner cannula (10) can be adjusted to different axial and angle positions relative to the outer cannula.

6. Tracheostomy cannula according to one of the preceding claims, **characterized in that** the inner cannula can be fixed relative to the outer cannula at different axial and different angular positions.

7. Tracheostomy cannula according to one of the preceding claims, **characterized in that** the inner cannula is adjustable in the axial direction and in the circumferential direction by means of a thread in the outer cannula.

8. Tracheostomy cannula according to claim 6, **characterized in that** an external thread is provided at the proximal end of the outer cannula, while the proximal end of the inner cannula comprises a flange which is held by a union nut which is in engagement with the external thread on which the outer cannula is held.

9. Tracheostomy cannula according to claim 6, **characterized in that** an external or internal thread, respectively, is provided at the proximal end of the inner cannula and the outer cannula, which are engagable with each other.

10. Tracheostomy cannula according to one of the preceding claims, **characterized in that** at the distal end of the outer cannula a stop for the distal end of the inner cannula is provided.

11. Tracheostomy cannula according to one of the preceding claims, **characterized in that** the inner cannula proximally comprises a flange or an end, respectively, which is captured by a union nut and adjusted and fixed at the proximal end of the outer cannula.

12. Tracheostomy cannula according to one of the preceding claims, **characterized in that** the outer cannula comprises at least two windows at different circumferential positions of the cannula.

13. Tracheostomy cannula according to one of the preceding claims, **characterized in that** the windows provided on the outer cannula and the inner cannula are designed in such a way, and the axial position of the inner cannula relative to the outer cannula is adjustable in such a way that in a first axial position the windows of the inner and outer cannula do not overlap and in a different position overlap to an extent that sufficient air for speaking can escape through the overlapping windows.

## Revendications

1. Canule de trachéostomie composée d'une canule externe (1) et d'une canule interne (10) pouvant être insérée à l'intérieur de façon ajustée, **caractérisée en ce que** la longueur efficace de la canule externe (1) et de la canule interne (10) est variable, la canule externe (1) et la canule interne (10) présentant au moins chacune une fenêtre (5, 9), les fenêtres (5, 9) se chevauchant au moins partiellement dans une direction axiale lorsque la canule interne (10) est insérée de façon ajustée dans la canule externe (1), la canule externe présentant une extrémité proximale qui comporte une plaque mobile.

2. Canule de trachéostomie selon la revendication 1, **caractérisée en ce que** la canule externe présente un segment facultativement extensible.

3. Canule de trachéostomie selon l'une des revendications 1 à 2, **caractérisée en ce que** la canule interne (10) peut pivoter par rapport à la canule externe (1).

4. Canule de trachéostomie selon l'une des revendications précédentes, **caractérisée en ce que** la fenêtre de la canule interne est exécutée pour être plus longue dans la direction axiale que la fenêtre de la canule externe.

5. Canule de trachéostomie selon l'une des revendications précédentes, **caractérisée en ce que** la canule interne (10) peut être réglée par rapport à la canule externe dans différentes positions axiales et angulaires.

6. Canule de trachéostomie selon l'une des revendications précédentes, **caractérisée en ce que** la canule interne peut être fixée par rapport à la canule externe dans différentes positions axiales et différentes positions angulaires.

7. Canule de trachéostomie selon l'une des revendications précédentes, **caractérisée en ce que** la canule interne peut être réglée dans la direction axiale et dans la direction périphérique via un filetage dans la canule externe.

8. Canule de trachéostomie selon la revendication 6, **caractérisée en ce qu'**un filetage externe est prévu au niveau de l'extrémité proximale de la canule externe tandis que l'extrémité proximale de la canule interne présente une bride qui est maintenue à la canule externe par un écrou d'accouplement qui se trouve en prise avec le filetage externe.

9. Canule de trachéostomie selon la revendication 6, **caractérisée en ce que** des filetages externes ou internes sont respectivement prévus au niveau de l'extrémité proximale des canules interne et externe, lesquels peuvent être en prise l'un avec l'autre.

10. Canule de trachéostomie selon l'une des revendications précédentes, **caractérisée en ce qu'**au niveau de l'extrémité distale de la canule externe est prévue une butée pour l'extrémité distale de la canule interne.

11. Canule de trachéostomie selon l'une des revendications précédentes, **caractérisée en ce que** la canule interne présente au niveau proximal une bride ou une extrémité qui est prise dans un écrou d'accouplement et est réglée et fixée au niveau de l'extrémité proximale de la canule externe.

12. Canule de trachéostomie selon l'une des revendications précédentes, **caractérisée en ce que** la canule externe présente au moins deux fenêtres au niveau de positions périphériques différentes de la canule.

13. Canule de trachéostomie selon l'une des revendications précédentes, **caractérisée en ce que** les fenêtres prévues au niveau de la canule externe et de la canule interne sont exécutées de façon telle et **en ce que** la position axiale de la canule interne peut être réglée de façon variable par rapport à la canule externe de façon telle que dans une première position axiale, les fenêtres des canules interne et externe ne se chevauchent pas, et se chevauchent dans une autre position de sorte que les fenêtres en position de chevauchement permettent un échange d'air suffisant pour parler.
